# FASCICULE DE BREVET EUROPEEN

(11) **EP 2 794 913 B1**
(45) Date de publication et mention de la délivrance du brevet: **20.09.2017**
(21) Numéro de dépôt: 12810390.0
(22) Date de dépôt: 17.12.2012
(51) Int. Cl.: C12Q 1/68

(54) **PROCEDE POUR LE DIAGNOSTIC IN VITRO DU CANCER DU COLON**
VERFAHREN ZUR IN-VITRO-DIAGNOSE VON DARMKREBS
METHOD FOR IN VITRO DIAGNOSIS OF COLON CANCER

(30) Priorité: 20.12.2011 FR 1162049
(43) Date de publication de la demande: 29.10.2014
(73) Titulaire: Biomérieux, 69280 Marcy l'Étoile (FR); Hospices Civils de Lyon, 69002 Lyon (FR)
(72) Inventeur: MALLET, François, 69100 Villeurbanne (FR); MUGNIER, Nathalie, 69008 Lyon (FR); PEROT, Philippe, 16710 Saint-Yrieix (FR)
(74) Mandataire: Agasse, Stéphane
(86) Numéro de dépôt international: PCT/FR2012/052954
(87) Numéro de publication internationale: WO 2013/093315

(56) Documents cités:
- EP-A1- 1 338 606
- EP-A1- 2 340 851
- WO-A1-2011/153684
- WENTZENSEN NICOLAS ET AL: "Identification of differentially expressed genes in colorectal adenoma compared to normal tissue by suppression subtractive hybridization.", INTERNATIONAL JOURNAL OF ONCOLOGY APR 2004 LNKD- PUBMED:15010839, vol. 24, no. 4, avril 2004 (2004-04), pages 987-994, XP009161875, ISSN: 1019-6439
- ALVES PEDRO M S ET AL: "Identification of tumor-associated antigens by large-scale analysis of genes expressed in human colorectal cancer.", CANCER IMMUNITY, vol. 8, 2008, pages 1-11, XP002681590, ISSN: 1424-9634
- J. GIMENEZ ET AL: "Custom human endogenous retroviruses dedicated microarray identifies self-induced HERV-W family elements reactivated in testicular cancer upon methylation control", NUCLEIC ACIDS RESEARCH, vol. 38, no. 7, 1 avril 2010 (2010-04-01), pages 2229-2246, XP055055471, ISSN: 0305-1048, DOI: 10.1093/nar/gkp1214
- KUNG AHN ET AL: "Structural and quantitative expression analyses of HERV gene family in human tissues", MOLECULES AND CELLS, vol. 28, no. 2, 1 août 2009 (2009-08-01), pages 99-103, XP055018135, ISSN: 1016-8478, DOI: 10.1007/s10059-009-0107-y
- LIANG QIAOYI ET AL: "Identification and detection of a novel human endogenous retrovirus-related gene, and structural characterization of its related elements", GENETICS AND MOLECULAR BIOLOGY, vol. 32, no. 4, 2009, page 704, XP002694344, ISSN: 1415-4757
- DATABASE EMBL [Online] 21 février 2002 (2002-02-21), "Homo sapiens chromosome 5 clone RP11-520E18, complete sequence.", XP002694332, extrait de EBI accession no. EM_STD:AC110721 Database accession no. AC110721
- J. PACES: "HERVd: the Human Endogenous RetroViruses Database: update", NUCLEIC ACIDS RESEARCH, vol. 32, no. 90001, 1 janvier 2004 (2004-01-01), pages 50D-50, XP055056305, ISSN: 0305-1048, DOI: 10.1093/nar/gkh075
- DATABASE EMBL [Online] 2 janvier 2007 (2007-01-02), "Homo sapiens endogenous virus HERV-H mRNA.", XP002681591, extrait de EBI accession no. EM_HUM:EF194101 Database accession no. EF194101 -& LIANG Q ET AL: "Identification of a novel human endogenous retrovirus and promoter activity of its 5' U3", BIOCHEMICAL AND BIOPHYSICAL RESEARCH COMMUNICATIONS, ACADEMIC PRESS INC. ORLANDO, FL, US, vol. 382, no. 2, 1 mai 2009 (2009-05-01), pages 468-472, XP026106790, ISSN: 0006-291X, DOI: 10.1016/J.BBRC.2009.03.058 [extrait le 2009-03-14]
- DATABASE EMBL [Online] 28 août 2000 (2000-08-28), "Homo sapiens Xp BAC RP11-315B16 (Roswell Park Cancer Institute Human BAC Library) complete sequence.", XP002698154, extrait de EBI accession no. EM_STD:AC079264 Database accession no. AC079264
- DATABASE EMBL [Online] 5 octobre 1999 (1999-10-05), "Human DNA sequence from clone RP1-122P22 on chromosome 20", XP002698155, extrait de EBI accession no. EM_STD:AL121761 Database accession no. AL121761
- DATABASE EMBL [Online] 28 janvier 2000 (2000-01-28), "Homo sapiens BAC clone RP11-563E2 from 4, complete sequence.", XP002698156, extrait de EBI accession no. EM_STD:AC022272 Database accession no. AC022272
- DATABASE EMBL [Online] 11 décembre 1998 (1998-12-11), "Homo sapiens PAC clone RP4-693M11 from 14q24.3, complete sequence.", XP002698157, extrait de EBI accession no. EM_STD:AC006146 Database accession no. AC006146
- T. Vinogradova ET AL: "Selective Differential Display of RNAs containing interspersed repeats: analysis of changes in the transcription of HERV-K LTRs in germ cell tumors", MGG - MOLECULAR GENETICS AND GENOMICS., vol. 266, no. 5, 1 January 2002 (2002-01-01), pages 796-805, XP055232494, DE ISSN: 1617-4615, DOI: 10.1007/s00438-001-0596-7
- PHILIPPE PÉROT ET AL: "Microarray-Based Sketches of the HERV Transcriptome Landscape", PLOS ONE, vol. 7, no. 6, 28 June 2012 (2012-06-28), page e40194, XP055034857, ISSN: 1932-6203, DOI: 10.1371/journal.pone.0040194
- PHILIPPE PÉROT ET AL: "Microarray-based sketches of the HERV transcriptome landscape: Supplementary Table S3", PLOS ONE, vol. 7, no. 6, 28 June 2012 (2012-06-28), XP055356742,

## Description

Les rétrovirus endogènes constituent la descendance de rétrovirus infectieux s'étant intégrés sous leur forme provirale dans des cellules de la lignée germinale et ayant été transmis par ce biais dans le génome de la descendance de l'hôte.

Le séquençage du génome humain a permis de révéler l'extrême abondance des éléments transposables ou de leurs dérivés. De fait, les séquences répétées représentent près de la moitié du génome humain et les rétrovirus endogènes et les rétrotransposons en composent 8% avec un nombre s'élevant, à ce jour, à plus de 400 000 éléments.

L'abondance des éléments rétroviraux endogènes (ERVs) présents actuellement dans le génome humain est le résultat d'une centaine d'endogénisations réussies au cours de l'évolution de la lignée humaine. Les différentes vagues d'endogénisation s'étalent sur une période allant de 2 à 90 millions d'années avant notre ère et ont été suivies de l'expansion du nombre de copies par des phénomènes de type « copier/coller » avec possibilité d'apparition d'erreurs, conduisant à partir d'un provirus ancestral à la formation d'une famille de HERV, c'est à dire un ensemble d'éléments présentant des homologies de séquences. Les plus anciens éléments, ceux de la famille HERV-L, se seraient intégrés avant l'émergence des mammifères. Deux familles HERV-F et HERV-H sont apparues dans la période où les premiers primates faisaient leur apparition. Les familles HERV-FRD et HERV-K(HML-5) intégrées il y a 40 à 55 millions d'années, sont spécifiques des primates supérieurs. En revanche les familles HERV-W et HERV-E, par exemple, se sont intégrées 5 à 10 millions d'années plus tard après la séparation avec les singes du nouveau monde, et sont spécifiques des Catarhini (Hominoides et Cercopithèques).

Les séquences ERVs sont représentées sur l'ensemble des chromosomes, avec une densité variant selon les familles et il n'existe pas de corrélation entre la proximité physique des ERVs et leur proximité phylogénétique.

Les ERVs ont longtemps été considérés comme des parasites ou comme de simples déchets de l'ADN. Néanmoins, l'impact des ERVs sur l'organisme n'est pas uniquement limité à leur participation passée dans le modelage du génome ou à des recombinaisons délétères pouvant encore subvenir.

L'abondance et la complexité structurelle des ERVs rendent les analyses de leur expression très compliquées et souvent difficilement interprétables. La détection de l'expression de HERV peut refléter l'activation transcriptionnelle de un ou de plusieurs loci au sein d'une même famille. Le ou les loci activés peuvent de plus varier en fonction du tissu et/ou du contexte.

Les présents inventeurs ont maintenant découvert et démontré, que des séquences d'acides nucléiques correspondant à des loci précisément identifiés d'éléments rétroviraux endogènes, sont associés au cancer du côlon et que ces séquences sont des marqueurs moléculaires de la pathologie. Les séquences identifiées sont soit des provirus, c'est à dire des séquences contenant tout ou partie des gènes gag, pol et env flanqués en 5' et 3' de longues terminaisons répétées (LTR ou « Long Terminal Repeat » selon la terminologie anglo-saxonne), soit tout ou partie des LTR ou des gènes isolés. Les séquences ADN identifiées sont respectivement référencées en SEQ ID NOs : 1 à 285 dans le listage de séquences, leur localisation chromosomique est identifiée dans le tableau ci-dessous (NCBI 36/hg18), ainsi que leur expression, surexpression ou sous-expression représentées par le « ratio d'expression » entre échantillon cancéreux et échantillon normal. Quand l'expression de l'acide nucléique ou le changement d'expression de l'acide nucléique est spécifique du tissu côlon on indique cette information par le symbole « x » dans la colonne tissu cible. Ceci signifie que si une expression ou un changement d'expression de l'acide nucléique concerné est déterminé dans un compartiment biologique autre que le tissu côlon, ceci représente, à distance, une signature d'un cancer du côlon. Les séquences ADN identifiées comme étant spécifiques du tissu côlon sont respectivement référencées en SEQ ID NOs : 1, 2, 3, 4, 5, 7, 8, 9, 10, 11, 12, 13, 17, 19, 22, 24, 25, 27 et 97 dans le listage de séquences. Les séquences ADN identifiées comme étant non spécifiques du tissu côlon sont respectivement référencées en SEQ ID NOs : 6, 14, 15, 16, 18, 20, 21, 23, 26, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284 et 285.

**Tableau**

| SEQ ID NO: | Localisation chromosomique | Tissu cible | Ratio d'expression cancer/normal |
|---|---|---|---|
| 1 | (-) chr 5: 135904531-135912105 | x | 43,8 |
| 2 | (-) chr X: 4468515-4474361 | x | 34,7 |
| 3 | (-) chr 20: 19680691-19685420 | x | 19,4 |
| 4 | (+) chr 4: 183972457-183977066 | x | 12,5 |
| 5 | (+) chr 13: 108715439-108721465 | x | 10,9 |
| 6 | (-) chr 1: 204451670-204454441 | | -8,6 |
| 7 | (-) chr 7: 26030345-26035880 | x | 8,4 |
| 8 | (+) chr 14: 73239795-73245370 | x | 8,3 |
| 9 | (-) chr 5: 179775292-179781337 | x | 7,7 |
| 10 | (-) chr 14: 30785319-30790095 | x | 7,6 |
| 11 | (-) chr 3: 45773285-45774220 | x | 6,7 |
| 12 | (+) chr 16: 84869202-84872386 | x | 6,4 |
| 13 | (-) chr 11: 130128601-130134242 | x | 5,9 |
| 14 | (-) chr 10: 6711545-6717509 | | 5,7 |
| 15 | (+) chr 8: 86591572-86592049 | | -5,6 |
| 16 | (+) chr 12: 25207414-25213718 | | 5,6 |
| 17 | (+) chr 8: 98256433-98262143 | x | 4,9 |
| 18 | (+) chr 1: 201726731-201727141 | | -4,8 |
| 19 | (-) chr 8: 80460894-80463081 | x | 4,8 |
| 20 | (+) chr 8: 105367316-105373215 | | 4,8 |
| 21 | (+) chr 17: 11815798-11820989 | | 4,7 |
| 22 | (-) chr 18: 24526989-24532843 | x | 4,5 |
| 23 | (+) chr 22: 15472270-15476039 | | 4,2 |
| 24 | (-) chr 8: 91171628-91177272 | x | 4,2 |
| 25 | (+) chr 1: 176275999-176281686 | x | 4,2 |
| 26 | (-) chr 19: 48519503-48525376 | | 4,1 |
| 27 | (+) chr 9: 25658927-25666384 | x | 3,7 |
| 28 | (+) chr 13: 34271392-34275485 | | 3,7 |
| 29 | (-) chr 11: 60237235-60238528 | | -3,6 |
| 30 | (+) chr 8: 116896352-116900252 | | 3,2 |
| 31 | (+) chr 19: 60146916-60147844 | | 3,2 |
| 32 | (+) chr 2: 188084458-188084785 | | -3,1 |
| 33 | (+) chr 10: 98482752-98486365 | | 2,9 |
| 34 | (-) chr 8: 99671725-99672015 | | 2,9 |
| 35 | (-) chr 13: 55586741-55590394 | | 2,8 |
| 36 | (-) chr X: 93840473-93846744 | | -2,8 |
| 37 | (-) chr 4: 93411664-93417822 | | 2,5 |
| 38 | (+) chr 4: 121134267-121140271 | | 2,5 |
| 39 | (-) chr 5: 135912105-135915610 | | 2,5 |
| 40 | (-) chr 6: 82110364-82117596 | | -2,4 |
| 41 | (-) chr 6: 123945178-123950851 | | 2,4 |
| 42 | (-) chr 2: 71238414-71246247 | | 2,4 |
| 43 | (-) chr 1: 171684177-171684627 | | -2,3 |
| 44 | (+) chr 10: 65936583-65936972 | | 2,3 |
| 45 | (+) chr 14: 50429630-50433350 | | 2,3 |
| 46 | (+) chr 14: 41054824-41060836 | | 2,3 |
| 47 | (-) chr 5: 34514678-34514916 | | 2,2 |
| 48 | (+) chr 8: 115366935-115369140 | | 2,2 |
| 49 | (-) chr 11: 73926652-73927082 | | 2,2 |
| 50 | (-) chr 14: 22736836-22737207 | | 2,2 |
| 51 | (-) chr 1: 79726879-79732396 | | 2,2 |
| 52 | (+) chr 3: 130161351-130164778 | | 2,1 |
| 53 | (-) chr 12: 11656097-11659027 | | -2,1 |
| 54 | (-) chr 17: 72113663-72119188 | | 2,1 |
| 55 | (-) chr 11: 123241237-123246982 | | 2,1 |
| 56 | (-) chr 8: 136848614-136854437 | | 2,1 |
| 57 | (+) chr 3: 135222763-135223084 | | -2,1 |
| 58 | (+) chr 12: 94666771-94672799 | | 2,1 |
| 59 | (+) chr 6: 63559535-63565316 | | -2,1 |
| 60 | (-) chr 2: 54587807-54590183 | | -2,0 |
| 61 | (+) chr 2: 173115073-173117251 | | 2,0 |
| 62 | (+) chr 5: 755586-759445 | | 2,0 |
| 63 | (+) chr 8: 129995887-130001750 | | 2,0 |
| 64 | (+) chr 4: 127503905-127504662 | | 2,0 |
| 65 | (-) chr 21: 41718929-41719369 | | 2,0 |
| 66 | (+) chr 20: 40489715-40490155 | | 2,0 |
| 67 | (+) chr 7: 100274888-100276065 | | 2,0 |
| 68 | (+) chr 7: 35702274-35703153 | | 2,0 |
| 69 | (+) chr 6: 131686129-131689771 | | -2,0 |
| 70 | (-) chr 1: 226879232-226884835 | | 2,0 |
| 71 | (-) chr 10: 17712042-17714165 | | 1,9 |
| 72 | (-) chr 10: 53463521-53469327 | | 1,9 |
| 73 | (-) chr X: 112238600-112244308 | | 1,9 |
| 74 | (-) chr 1: 146722974-146723946 | | 1,9 |
| 75 | (+) chr 1: 53671142-53673436 | | 1,9 |
| 76 | (-) chr X: 91414738-91420449 | | -1,9 |
| 77 | (-) chr 7: 16683220-16684043 | | 1,9 |
| 78 | (+) chr 10: 100097920-100098685 | | 1,9 |
| 79 | (+) chr 22: 31228741-31234490 | | 1,8 |
| 80 | (-) chr 13: 112501931-112502014 | | 1,8 |
| 81 | (+) chr 1: 146832410-146833382 | | 1,8 |
| 82 | (-) chr 10: 20449793-20453869 | | 1,8 |
| 83 | (+) chr 9: 21911892-21921934 | | 1,8 |
| 84 | (-) chr 3: 186574589-186580188 | | -1,8 |
| 85 | (-) chr 10: 62463482-62469677 | | -1,8 |
| 86 | (-) chr 15: 97959745-97960384 | | -1,7 |
| 87 | (+) chr 17: 75988189-75996283 | | -1,7 |
| 88 | (+) chr 2: 88760597-88762729 | | 1,7 |
| 89 | (+) chr 7: 29791133-29794522 | | 1,7 |
| 90 | (+) chr 18: 50528195-50528890 | | -1,7 |
| 91 | (+) chr 1: 89162808-89170138 | | -1,7 |
| 92 | (-) chr 3: 178866314-178871614 | | -1,7 |
| 93 | (-) chr 19: 52245264-52250506 | | 1,7 |
| 94 | (+) chr 17: 30852752-30853047 | | -1,7 |
| 95 | (-) chr 11: 69581214-69582655 | | 1,7 |
| 96 | (+) chr 5: 138886787-138888726 | | -1,7 |
| 97 | (-) chr 3: 167701085-167706900 | x | 1,7 |
| 98 | (+) chr 1: 101404629-101404696 | | 1,7 |
| 99 | (+) chr 8: 113277279-113282810 | | -1,6 |
| 100 | (-) chr 20: 740491-741481 | | 1,6 |
| 101 | (-) chr 2: 58194071-58199769 | | 1,6 |
| 102 | (+) chr 13: 35788462-35794330 | | -1,6 |
| 103 | (+) chr 9: 12938344-12944129 | | 1,6 |
| 104 | (-) chr 9: 116976750-116982362 | | -1,6 |
| 105 | (-) chr 4: 11264289-11269976 | | -1,6 |
| 106 | (-) chr 1: 223094264-223100407 | | -1,6 |
| 107 | (+) chr 7: 92862465-92866875 | | 1,6 |
| 108 | (-) chr 17: 72296838-72297401 | | -1,6 |
| 109 | (+) chr X: 86823336-86830473 | | 1,6 |
| 110 | (-) chr X: 89718079-89718557 | | -1,6 |
| 111 | (-) chr 3: 58397972-58398349 | | -1,6 |
| 112 | (-) chr 2: 215413351-215413804 | | -1,6 |
| 113 | (+) chr 4: 87614948-87620699 | | 1,6 |
| 114 | (-) chr 1: 54874382-54875358 | | -1,6 |
| 115 | (+) chr 1: 237429952-237430254 | | 1,6 |
| 116 | (-) chr 7: 79651365-79652053 | | -1,6 |
| 117 | (-) chr 4: 180711319-180712287 | | -1,5 |
| 118 | (-) chr 5: 146727162-146727562 | | -1,5 |
| 119 | (+) chr 3: 156178559-156179784 | | -1,5 |
| 120 | (+) chr 1: 51465235-51468757 | | -1,5 |
| 121 | (-) chr 3: 100178327-100178459 | | 1,5 |
| 122 | (-) chr 4: 67932305-67932737 | | 1,5 |
| 123 | (-) chr 18: 24532993-24533490 | | 1,5 |
| 124 | (-) chr 12: 20862483-20866818 | | 1,5 |
| 125 | (-) chr 12: 8334466-8337953 | | -1,5 |
| 126 | (-) chr 13: 94759022-94759378 | | -1,5 |
| 127 | (-) chr 1: 144779633-144780605 | | 1,5 |
| 128 | (+) chr 1: 143314771-143315743 | | 1,5 |
| 129 | (+) chr 2: 231645891-231645949 | | 1,5 |
| 130 | (-) chr 7: 65721380-65722348 | | -1,5 |
| 131 | (+) chr 2: 188606470-188612115 | | -1,5 |
| 132 | (-) chr 3: 176879333-176879730 | | -1,5 |
| 133 | (-) chr 3: 186765708-186772209 | | 1,5 |
| 134 | (-) chr 11: 67377518-67381008 | | 1,5 |
| 135 | (-) chr 12: 123157590-123158004 | | 1,5 |
| 136 | (+) chr 7: 134507781-134510522 | | -1,5 |
| 137 | (+) chr 12: 112980007-112980079 | | -1,5 |
| 138 | (-) chr 10: 101571639-101577563 | | 1,5 |
| 139 | (+) chr 8: 81814506-81817982 | | -1,5 |
| 140 | (-) chr 12: 29178820-29185001 | | -1,5 |
| 141 | (-) chr 5: 119558433-119559245 | | -1,5 |
| 142 | (-) chr 13: 23891789-23892035 | | 1,5 |
| 143 | (-) chr 1: 51845967-51846990 | | -1,5 |
| 144 | (-) chr 19: 58630272-58630359 | | 1,5 |
| 145 | (-) chr 3: 193273756-193277341 | | -1,5 |
| 146 | (+) chr 6: 149002601-149008339 | | -1,5 |
| 147 | (+) chr 3: 186986788-186987217 | | 1,5 |
| 148 | (-) chr 3: 192866741-192872861 | | 1,5 |
| 149 | (+) chr 4: 69952306-69955060 | | -1,4 |
| 150 | (-) chr 14: 38837505-38837576 | | 1,4 |
| 151 | (+) chr 2: 57102769-57103266 | | -1,4 |
| 152 | (-) chr 3: 130032422-130036475 | | -1,4 |
| 153 | (+) chr 4: 54112155-54112817 | | -1,4 |
| 154 | (-) chr 3: 146311752-146313676 | | 1,4 |
| 155 | (-) chr 12: 14769094-14769447 | | 1,4 |
| 156 | (+) chr 13: 55512386-55518412 | | 1,4 |
| 157 | (+) chr 15: 72433538-72439180 | | -1,4 |
| 158 | (+) chr 3: 40577391-40583175 | | -1,4 |
| 159 | (-) chr 4: 120675771-120676048 | | -1,4 |
| 160 | (-) chr 8: 12395268-12398823 | | -1,4 |
| 161 | (-) chr X: 101106329-101106710 | | 1,4 |
| 162 | (-) chr 3: 190138663-190139459 | | -1,4 |
| 163 | (-) chr 3: 98473995-98479084 | | -1,4 |
| 164 | (+) chr 10: 5070454-5070498 | | -1,4 |
| 165 | (+) chr 5: 114710170-114711150 | | 1,4 |
| 166 | (+) chr 3: 128381736-128382403 | | -1,4 |
| 167 | (+) chr 9: 22813029-22813838 | | -1,4 |
| 168 | (-) chr 2: 231673651-231673769 | | 1,4 |
| 169 | (+) chr 3: 116946112-116948448 | | 1,4 |
| 170 | (-) chr 11: 92482373-92482685 | | 1,4 |
| 171 | (+) chr 4: 167867005-167868159 | | 1,4 |
| 172 | (-) chr 1: 144779633-144780605 | | 1,4 |
| 173 | (+) chr 20: 58157372-58158054 | | 1,4 |
| 174 | (-) chr 4: 138339335-138340015 | | -1,4 |
| 175 | (-) chr 12: 71688770-71689742 | | 1,4 |
| 176 | (+) chr 13: 63310225-63315979 | | -1,4 |
| 177 | (-) chr 1: 110201971-110202287 | | -1,4 |
| 178 | (+) chr 10: 92557026-92562997 | | -1,4 |
| 179 | (+) chr 1: 38146074-38152175 | | -1,4 |
| 180 | (+) chr 6: 153045773-153046224 | | 1,4 |
| 181 | (-) chr 1: 79934615-79940335 | | -1,4 |
| 182 | (+) chr 4: 155197148-155204405 | | -1,4 |
| 183 | (+) chr 13: 40927858-40928571 | | 1,4 |
| 184 | (+) chr 4: 11974611-11980403 | | -1,3 |
| 185 | (+) chr 18: 4676671-4682409 | | -1,3 |
| 186 | (-) chr 21: 14575132-14576104 | | -1,3 |
| 187 | (+) chr 5: 180186875-180187838 | | -1,3 |
| 188 | (+) chr 4: 68579259-68585888 | | -1,3 |
| 189 | (-) chr 11: 27607193-27613049 | | -1,3 |
| 190 | (+) chr 18: 6123610-6123972 | | -1,3 |
| 191 | (+) chr 14: 57658752-57662615 | | -1,3 |
| 192 | (-) chr 1: 144213068-144214036 | | -1,3 |
| 193 | (-) chr 4: 62312529-62318338 | | -1,3 |
| 194 | (+) chr 1: 45751770-45752724 | | -1,3 |
| 195 | (-) chr 17: 6577008-6581495 | | -1,3 |
| 196 | (-) chr 1: 244819996-244820439 | | -1,3 |
| 197 | (-) chr 9: 100067414-100073409 | | 1,3 |
| 198 | (+) chr 3: 196356095-196356926 | | -1,3 |
| 199 | (+) chr 12: 31851416-31851846 | | -1,3 |
| 200 | (-) chr 5: 118311697-118316179 | | -1,3 |
| 201 | (-) chr 1: 94806343-94810970 | | -1,3 |
| 202 | (+) chr 2: 185332604-185332980 | | -1,3 |
| 203 | (+) chr 4: 107250443-107255274 | | -1,3 |
| 204 | (-) chr 18: 31472178-31472680 | | -1,3 |
| 205 | (+) chr 6: 38501905-38502660 | | -1,3 |
| 206 | (-) chr 15: 49734758-49735530 | | 1,3 |
| 207 | (-) chr X: 84258326-84258441 | | -1,3 |
| 208 | (+) chr 5: 55618832-55619003 | | -1,3 |
| 209 | (-) chr 17: 16678919-16679896 | | 1,3 |
| 210 | (+) chr 2: 193715387-193716358 | | -1,3 |
| 211 | (+) chr 5: 115014416-115019003 | | 1,3 |
| 212 | (+) chr 17: 22039285-22039750 | | -1,3 |
| 213 | (+) chr 5: 108685641-108685853 | | -1,3 |
| 214 | (+) chr 10: 65474521-65475382 | | -1,3 |
| 215 | (-) chr 6: 89180631-89187940 | | 1,3 |
| 216 | (+) chr 1: 244312610-244313561 | | 1,3 |
| 217 | (+) chr 5: 76822592-76823033 | | -1,3 |
| 218 | (-) chr Y: 4283432-4289271 | | -1,3 |
| 219 | (+) chr 5: 71075676-71076376 | | -1,3 |
| 220 | (+) chr 2: 24861412-24861769 | | -1,3 |
| 221 | (-) chr 1: 110196377-110201971 | | -1,3 |
| 222 | (+) chr 3: 177261846-177267467 | | -1,3 |
| 223 | (+) chr 7: 6908153-6909119 | | -1,3 |
| 224 | (-) chr 12: 33132053-33135058 | | -1,3 |
| 225 | (-) chr 7: 146321019-146321687 | | 1,3 |
| 226 | (+) chr 4: 54581003-54585874 | | -1,3 |
| 227 | (+) chr X: 138823266-138823672 | | -1,3 |
| 228 | (+) chr 18: 2829330-2829995 | | -1,3 |
| 229 | (+) chr 6: 66335631-66336029 | | -1,3 |
| 230 | (-) chr 6: 121983265-121989145 | | -1,3 |
| 231 | (-) chr 2: 226039745-226040459 | | -1,3 |
| 232 | (+) chr 4: 109853300-109855458 | | 1,3 |
| 233 | (+) chr 19: 20126744-20133095 | | -1,3 |
| 234 | (-) chr 11: 14839752-14840420 | | -1,2 |
| 235 | (-) chr 3: 20380459-20380671 | | -1,2 |
| 236 | (+) chr 4: 53395726-53396112 | | -1,2 |
| 237 | (+) chr 19: 9553879-9554314 | | 1,2 |
| 238 | (-) chr 2: 63928103-63928550 | | -1,2 |
| 239 | (+) chr 3: 187581597-187582311 | | -1,2 |
| 240 | (-) chr 5: 76211738-76218598 | | -1,2 |
| 241 | (+) chr 17: 18280923-18281897 | | 1,2 |
| 242 | (-) chr 4: 178566070-178566839 | | -1,2 |
| 243 | (+) chr 12: 60267154-60272981 | | -1,2 |
| 244 | (+) chr 18: 27623185-27623837 | | -1,2 |
| 245 | (-) chr 5: 135094585-135101074 | | -1,2 |
| 246 | (+) chr 10: 4962301-4962771 | | -1,2 |
| 247 | (-) chr 5: 153996685-154003250 | | -1,2 |
| 248 | (-) chr 6: 5981329-5981780 | | -1,2 |
| 249 | (-) chr 8: 828875-829287 | | -1,2 |
| 250 | (+) chr 18: 38295804-38303710 | | -1,2 |
| 251 | (-) chr 3: 54643549-54649271 | | -1,2 |
| 252 | (-) chr 18: 68050597-68051144 | | -1,2 |
| 253 | (-) chr 3: 46260865-46268560 | | -1,2 |
| 254 | (-) chr 21: 40033061-40040223 | | -1,2 |
| 255 | (+) chr 1: 148879269-148880889 | | 1,2 |
| 256 | (-) chr 2: 44343693-44344377 | | 1,2 |
| 257 | (-) chr 11: 80087903-80088662 | | -1,2 |
| 258 | (+) chr 3: 46140179-46140255 | | -1,2 |
| 259 | (+) chr 10: 45388242-45391541 | | -1,2 |
| 260 | (-) chr 10: 52210114-52210776 | | 1,2 |
| 261 | (-) chr 3: 163923299-163927462 | | -1,2 |
| 262 | (-) chr 18: 22761239-22762201 | | -1,2 |
| 263 | (+) chr 5: 64505763-64506756 | | -1,2 |
| 264 | (-) chr 16: 18909966-18916124 | | -1,2 |
| 265 | (+) chr 5: 119114744-119115548 | | -1,2 |
| 266 | (+) chr 1: 69101725-69102525 | | -1,2 |
| 267 | (+) chr 1: 161827723-161832945 | | -1,2 |
| 268 | (+) chr 1: 223140681-223147414 | | -1,2 |
| 269 | (+) chr Y: 26648032-26648192 | | -1,2 |
| 270 | (-) chr 2: 195907646-195913044 | | -1,2 |
| 271 | (-) chr 7: 54710388-54711318 | | -1,2 |
| 272 | (-) chr 8: 34837245-34837699 | | 1,2 |
| 273 | (+) chr 4: 27585609-27586295 | | -1,2 |
| 274 | (+) chr 6: 160569673-160575346 | | -1,2 |
| 275 | (-) chr 2: 167330763-167331232 | | -1,1 |
| 276 | (-) chr 21: 16686151-16692725 | | -1,1 |
| 277 | (-) chr 3: 180722967-180726830 | | -1,1 |
| 278 | (+) chr 13: 61603944-61604298 | | -1,1 |
| 279 | (-) chr Y: 15720288-15724345 | | -1,1 |
| 280 | (-) chr 1: 115355475-115355826 | | 1,1 |
| 281 | (+) chr Y: 26648032-26648192 | | -1,1 |
| 282 | (+) chr 11: 94682231-94690615 | | -1,1 |
| 283 | (-) chr 18: 62088302-62089092 | | 1,1 |
| 284 | (+) chr 19: 49834194-49835177 | | -1,1 |
| 285 | (-) chr 11: 69737702-69743910 | | -1,1 |

La présente invention a donc pour objet un procédé pour le diagnostic, *in vitro,* du cancer du côlon dans un échantillon biologique prélevé chez un patient, qui comprend une étape de détection d'au moins un produit d'expression d'au moins une séquence d'acide nucléique, ladite séquence d'acide nucléique étant choisie parmi les séquences identifiées en SEQ ID NOs : 1, 3, 4, 5, 7, 8, 9, 10, 11, 12, 13, 17, 19, 22, 24, 25, 27 et 97 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 3, 4, 5, 7, 8, 9, 10, 11, 12, 13, 17, 19, 22, 24, 25, 27 et 97.

Le diagnostic permet d'établir si un individu est malade ou non malade. Le pronostic permet d'établir un degré de gravité de la maladie (grades et/ou stades) qui a une incidence sur la survie et/ou qualité de vie de l'individu. Dans le cadre de la présente invention, le diagnostic peut être très précoce.

Le pourcentage d'identité décrit ci-dessus a été déterminé en prenant en considération la diversité nucléotidique dans le génome. Il est connu que la diversité nucléotidique est plus élevée dans les régions du génome riches en séquences répétées que dans les régions ne contenant pas de séquences répétées. A titre d'exemple, Nickerson D. A. et al. (1) ont montré une diversité d'environ 0,3% (0,32%) dans des régions contenant des séquences répétées.

La capacité de discrimination d'un état cancéreux de chacune des séquences identifiées ci-dessus a été mise en évidence à l'aide d'une analyse statistique utilisant la procédure SAM (5) suivie d'une correction par le taux de faux positif (6) et d'une
suppression des valeurs inférieures à 2⁶. Par conséquent chacune des séquences identifiées ci-dessus présente une différence d'expression significative entre un état tumoral et un état normal. Il en résulte qu'une différence d'expression observée pour une des séquences précitées constitue une signature de la pathologie. Selon l'invention, il est possible de combiner les différences d'expression relevées pour plusieurs des séquences référencées ci-dessus par exemple par une ou des associations de 2, 3, 4, 5, 6, 7, 8, 9, 10 voire plus et jusqu'à 285 des séquences listées, de préférence par une ou des associations de 2, 3, 4, 5, 6, 7, 8, 9, 10 voire plus et jusqu'à 18 des séquences respectivement identifiées en SEQ ID NOs : 1, 2, 3, 4, 5, 7, 8, 9, 10, 11, 12, 13, 17, 19, 22, 24, 25, 27 et 97 et celles qui présentent au moins 99% d'identité avec les séquences identifiées en SEQ ID NOs : 1, 2, 3, 4, 5, 7, 8, 9, 10, 11, 12, 13, 17, 19, 22, 24, 25, 27 et 97.

En particulier les séquences identifiées en SEQ ID NOs : 2, 3 et 8, prises seules ou en combinaison (2 à 2 ou 3) constituent une ou des signatures privilégiées.

Dans un mode de réalisation du procédé selon l'invention on détecte le produit d'expression d'au moins deux séquences d'acide nucléique, lesdites au moins deux séquences d'acide nucléique étant choisies parmi les séquences identifiées comme étant spécifiques du tissu côlon, c'est à dire choisies dans le groupe de séquences identifiées en SEQ ID NOs : 1, 2, 3, 4, 5, 7, 8, 9, 10, 11, 12, 13, 17, 19, 22, 24, 25, 27 et 97 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 2, 3, 4, 5, 7, 8, 9, 10, 11, 12, 13, 17, 19, 22, 24, 25, 27 et 97.

Dans un autre mode de réalisation du procédé de l'invention, on détecte le produit d'expression d'au moins une séquence choisie parmi les séquences identifiées comme étant spécifiques du tissu côlon, c'est à dire choisie dans le groupe de séquences identifiées en SEQ ID NOs : 1, 2, 3, 4, 5, 7, 8, 9, 10, 11, 12, 13, 17, 19, 22, 24, 25, 27 et 97 ou choisie parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 2, 3, 4, 5, 7, 8, 9, 10, 11, 12, 13, 17, 19, 22, 24, 25, 27 et 97 et le produit d'expression d'au moins une séquence choisie parmi les séquences identifiées comme étant non spécifiques du tissu côlon, c'est à dire choisie dans le groupe de séquences identifiées en SEQ ID NOs : 6, 14, 15, 16, 18, 20, 21, 23, 26, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284 et 285 ou choisie parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 6, 14, 15, 16, 18, 20, 21, 23, 26, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284 et 285.

En particulier, dans le procédé de l'invention on détecte le produit d'expression d'au moins une séquence d'acide nucléique, de préférence d'au moins deux séquences d'acide nucléique ou de trois séquences d'acide nucléique, lesdites séquences d'acide nucléique étant choisies dans le groupe de séquences identifiées en SEQ ID NOs : 2, 3 et 8, ou parmi les séquences qui présentent au moins 99% d'identité, de préférence au moins 99,5% d'identité et avantageusement au moins 99,6% ou au moins 99,7% d'identité avec les séquences identifiées en SEQ ID NOs : 2, 3 et 8.

Le produit d'expression détecté est au moins un transcrit ARN, en particulier au moins un ARNm ou au moins un polypeptide.

Lorsque le produit d'expression est un transcrit ARNm, il est détecté par toute méthode appropriée, telle que l'hybridation, le séquençage ou l'amplification. L'ARNm peut être détecté directement par mise en contact avec au moins une sonde et/ou au moins une amorce qui sont conçues pour s'hybrider dans des conditions expérimentales prédéterminées aux transcrits ARNm, la mise en évidence de la présence ou de l'absence d'hybridation à l'ARNm et éventuellement la quantification de l'ARNm. Parmi les méthodes préférées on peut citer l'amplification (par exemple la RT-PCR, la NASBA, etc.), l'hybridation sur puce ou encore le séquençage. L'ARNm peut également être détecté indirectement à partir d'acides nucléiques dérivés desdits transcrits, comme les copies ADNc, etc.

Généralement le procédé de l'invention comprend une étape initiale d'extraction de l'ARNm de l'échantillon à analyser.

Ainsi, le procédé peut comprendre :
(i) une étape d'extraction de l'ARNm de l'échantillon à analyser,
(ii) une étape de détection et de quantification de l'ARNm de l'échantillon à analyser,
(iii) une étape d'extraction de l'ARNm dans un échantillon de référence, qui peut être un échantillon sain et provenant du même individu ou,
(iv) une étape de détection et de quantification de l'ARNm de l'échantillon sain,
(iii) une étape de comparaison de la quantité d'ARNm exprimé dans l'échantillon à analyser et dans l'échantillon de référence ; la détermination d'une quantité d'ARNm exprimé dans l'échantillon à analyser différente de la quantité d'ARNm exprimé dans l'échantillon de référence sain pouvant être corrélée avec le diagnostic ou le pronostic de gravité d'un cancer du côlon (la différence de la quantité d'ARNm dans le tissu côlon cancéreux par rapport à la quantité d'ARNm exprimé dans le tissu côlon sain étant indifféremment une expression, une surexpression ou une sous-expression);
et en particulier :
(i) une extraction de l'ARNm à analyser de l'échantillon,
(ii) une détermination, dans l'ARN à analyser, d'un niveau d'expression d'au moins une séquence ARN dans l'échantillon, de préférence d'au moins deux séquences ARN dans l'échantillon, la séquence ARN et les séquences ARN étant respectivement le produit de transcription d'au moins une séquence d'acide nucléique et d'au moins deux séquences d'acides nucléiques choisies parmi les séquences identifiéesprécédemment, et
(iii) une comparaison du niveau d'expression de la ou des séquence(s) ARN définie(s) en (ii) avec un niveau d'expression de référence ; la détermination d'un niveau d'expression de l'ARN à analyser présentant une différence par rapport au niveau d'expression de référence pouvant être corrélé avec le diagnostic ou le pronostic d'un cancer du côlon (comme déterminé ci-dessus) ; ou
(i) une étape d'extraction de l'ARNm de l'échantillon à analyser,
(ii) une étape de détection et de quantification de l'ARNm de l'échantillon à analyser,
(iii) une étape de comparaison de la quantité d'ARNm exprimé dans l'échantillon à analyser par rapport à une quantité d'ARNm de référence, la détermination d'une quantité d'ARNm exprimé dans l'échantillon à analyser différente de la quantité d'ARNm de référence pouvant être corrélée avec le diagnostic ou le pronostic d'un cancer du côlon (la différence de la quantité d'ARNm dans l'échantillon à analyser par rapport à la quantité d'ARNm de référence étant indifféremment une expression, une surexpression ou une sous-expression).

Dans un mode de réalisation du procédé de l'invention on prépare des copies ADN de l'ARNm, on met en contact les copies ADN avec au moins une sonde et/ou au moins une amorce dans des conditions prédéterminées permettant l'hybridation et en ce qu'on détecte la présence ou l'absence d'hybridation aux dites copies ADN.

Le produit d'expression qui est détecté peut aussi être un polypeptide qui est le produit de la traduction d'au moins un des transcrits décrits ci-dessus. Auquel cas, on détecte le polypeptide exprimé par mise en contact avec au moins un partenaire de liaison spécifique dudit polypeptide, en particulier un anticorps ou un analogue d'anticorps ou un aptamère. Le partenaire de liaison est de préférence un anticorps, par exemple un anticorps monoclonal ou un anticorps polyclonal hautement purifié ou un analogue d'un anticorps, par exemple une protéine d'affinité aux propriétés compétitives (nanofitine^{™}).

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Les nanofitines™ sont de petites protéines qui comme les anticorps sont capables de se lier à une cible biologique permettant ainsi de la détecter, de la capturer ou tout simplement de la cibler au sein d'un organisme. On les présente, entre autres, comme des analogues d'anticorps.

Les aptamères sont des oligonucléotides synthétiques capables de fixer un ligand spécifique.

L'invention concerne également l'utilisation d'au moins une séquence d'acide nucléique, isolée, comme marqueur moléculaire pour le diagnostic *in vitro* du cancer du côlon, caractérisée en ce que ladite séquence d'acide nucléique consiste en :
(i) au moins une séquence ADN choisie parmi les séquences SEQ ID NOs : 1, 3, 4, 5, 7, 8, 9, 10, 11, 12, 13, 17, 19, 22, 24, 25, 27 et 97, ou
(ii) au moins une séquence ADN complémentaire d'une séquence choisie parmi les séquences SEQ ID NOs : 1, , 3, 4, 5, 7, 8, 9, 10, 11, 12, 13, 17, 19, 22, 24, 25, 27 et 97, ou
(iii) au moins une séquence ADN qui présente au moins 99% d'identité, de préférence au moins 99,5% d'identité et avantageusement au moins 99,6% ou au moins 99,7% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité, de préférence au moins 99,5% d'identité et avantageusement au moins 99,6% ou au moins 99,7% d'identité avec une séquence telle que définie en (i).

Dans un mode de réalisation, on utilise au moins deux séquences d'acide nucléique qui consistent en :
(i) au moins deux séquences ADN choisies parmi les séquences identifiées en SEQ ID NOs : 1, 2, 3, 4, 5, 7, 8, 9, 10, 11, 12, 13, 17, 19, 22, 24, 25, 27 et 97 et en particulier les séquences SEQ ID Nos : 2, 3 et 8, ou
(ii) au moins deux séquences ADN respectivement complémentaires d'au moins deux séquences choisies parmi les séquences identifiées en SEQ ID NOs : 1, 2, 3, 4, 5, 7, 8, 9, 10, 11, 12, 13, 17, 19, 22, 24, 25, 27 et 97 et en particulier choisies parmi les séquences SEQ ID Nos : 2, 3 et 8, ou
(iii) au moins deux séquences ADN qui présentent respectivement au moins 99% d'identité, de préférence au moins 99,5% d'identité et avantageusement au moins 99,6% ou au moins 99,7% d'identité avec deux séquences telles que définies en (i) et (ii), ou
(iv) au moins deux séquences ARN qui sont respectivement le produit de transcription de deux séquences choisies parmi les séquences telles que définies en (i), ou
(v) au moins deux séquences ARN qui sont le produit de transcription de deux séquences choisies parmi les séquences qui présentent au moins 99% d'identité, de préférence au moins 99,5% d'identité et avantageusement au moins 99,6% ou au moins 99,7% d'identité avec les séquences telles que définies en (i).

L'invention concerne encore un procédé pour évaluer l'efficacité d'un traitement du cancer du côlon qui comprend un étape d'obtention d'une série d'échantillons biologiques, une étape de détection d'au moins un produit d'expression d'au moins une séquence d'acide nucléique dans ladite série d'échantillons biologiques, ladite séquence d'acide nucléique étant choisie parmi les séquences identifiées en SEQ ID NOs : 1 , 3, 4, 5, 7, 8, 9, 10, 11, 12, 13, 17, 19, 22, 24, 25, 27 et 97 ou parmi des séquences qui présentent au moins 99% d'identité, de préférence au moins 99,5% d'identité et avantageusement au moins 99,6% ou au moins 99,7% d'identité avec les séquences identifiées en SEQ ID NOs 1, 3, 4, 5, 7, 8, 9, 10, 11, 12, 13, 17, 19, 22, 24, 25, 27 et 97.

Dans un mode de réalisation, on détecte au moins deux produits d'expression d'au moins deux séquences d'acide nucléique, lesdites deux séquences d'acide nucléique étant choisies parmi les séquences identifiées en SEQ ID NOs : 1, 2, 3, 4, 5, 7, 8, 9, 10, 11, 12, 13, 17, 19, 22, 24, 25, 27 et 97 ou parmi les séquences qui présentent au moins 99% d'identité respectivement, de préférence au moins 99,5% d'identité et avantageusement au moins 99,6% ou au moins 99,7% d'identité avec les séquences identifiées en SEQ ID NOs : 1, 2, 3, 4, 5, 7, 8, 9, 10, 11, 12, 13, 17, 19, 22, 24, 25, 27 et 97.

Dans un autre mode de réalisation du procédé, on détecte le produit d'expression d'au moins une séquence d'acide nucléique, de préférence d'au moins deux séquences d'acides nucléiques ou de trois séquences d'acide nucléique, lesdites séquences d'acide nucléiques étant choisies dans le groupe de séquences identifiées en SEQ ID NOs 2, 3 et 8 ou parmi les séquences qui présentent au moins 99% d'identité respectivement, de préférence au moins 99,5% d'identité et avantageusement au moins 99,6% ou au moins 99,7% d'identité avec les séquences identifiées en SEQ ID NOs : 2, 3 et 8.

Par échantillon biologique on entend un tissu, un fluide, des composants desdits tissu et fluide, tels que des cellules ou des corps apoptotiques, des vésicules excrétées, comprenant notamment des exosomes et des microvésicules. A titre d'exemple, l'échantillon biologique peut être issu d'une biopsie du côlon réalisée au préalable chez un patient suspecté d'être atteint d'un cancer du côlon ou être issu d'une biopsie pratiquée sur un organe autre que le côlon chez un patient présentant des métastases. Dans ce deuxième cas, quand le changement d'expression de l'acide nucléique (marqueur moléculaire) est spécifique de l'organe côlon, il est possible de remonter au cancer primaire, c'est à dire au cancer du côlon. L'échantillon biologique peut être également un fluide biologique, tel que du sang ou une fraction sanguine (sérum, plasma), de l'urine, de la salive, du liquide céphalo-rachidien, de la lymphe, du lait maternel, du sperme, de même que des composants desdits fluides, en particulier des vésicules excrétées telles que définies ci-dessus. Par exemple, la détection d'un transcrit spécifique du tissu côlon dans un exosome ou une microvésicule, originaire d'une cellule épithéliale, signe soit la présence d'un cancer primaire, soit des métastases, sans qu'il soit nécessaire de faire un prélèvement au niveau de l'organe.

### Figures :

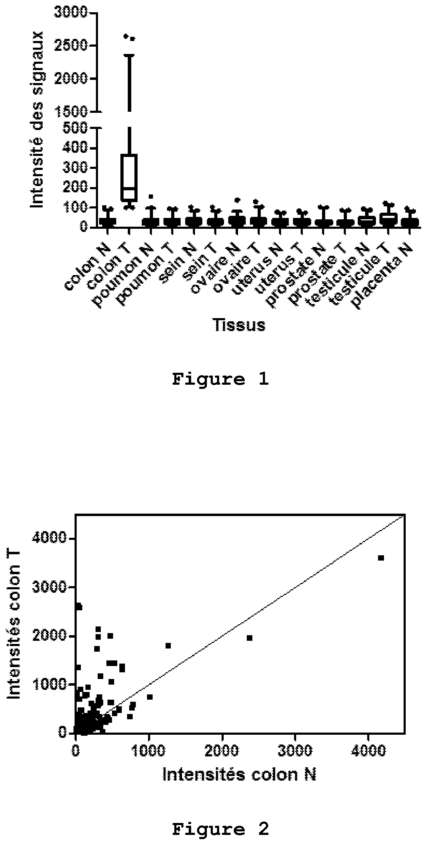

Les figures 1 et 2 représentent le différentiel d'expression observé dans le cancer du côlon pour un ensemble de séquences HERV. Plus précisément, la figure 1 (clustering) regroupe de manière exploratoire les éléments HERV qui ont un tropisme d'expression associé au cancer du côlon par rapport à l'ensemble des tissus contrôles, et la figure 2 montre les différences statistiques d'expression d'éléments HERV entre côlon normal et côlon tumoral.

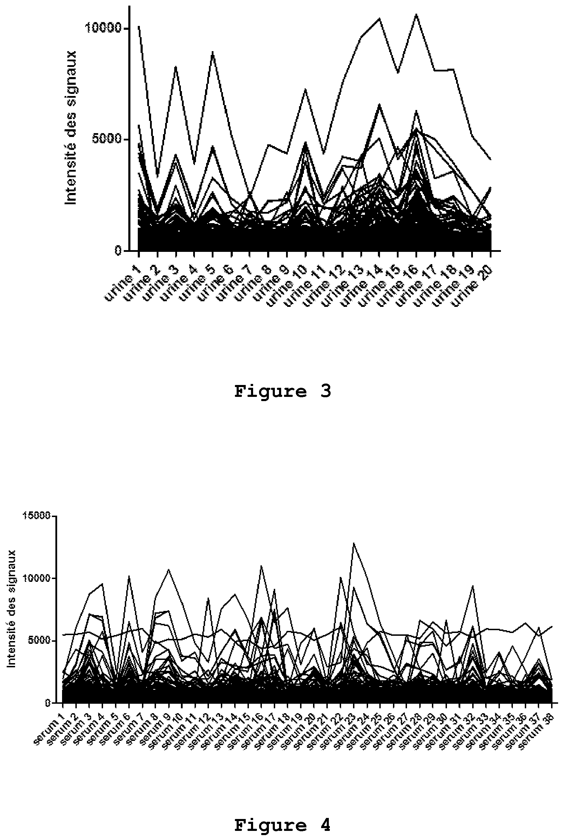

Les figures 3 et 4 montrent la détection de séquences HERV dans deux fluides biologiques : les urines et les sérums.

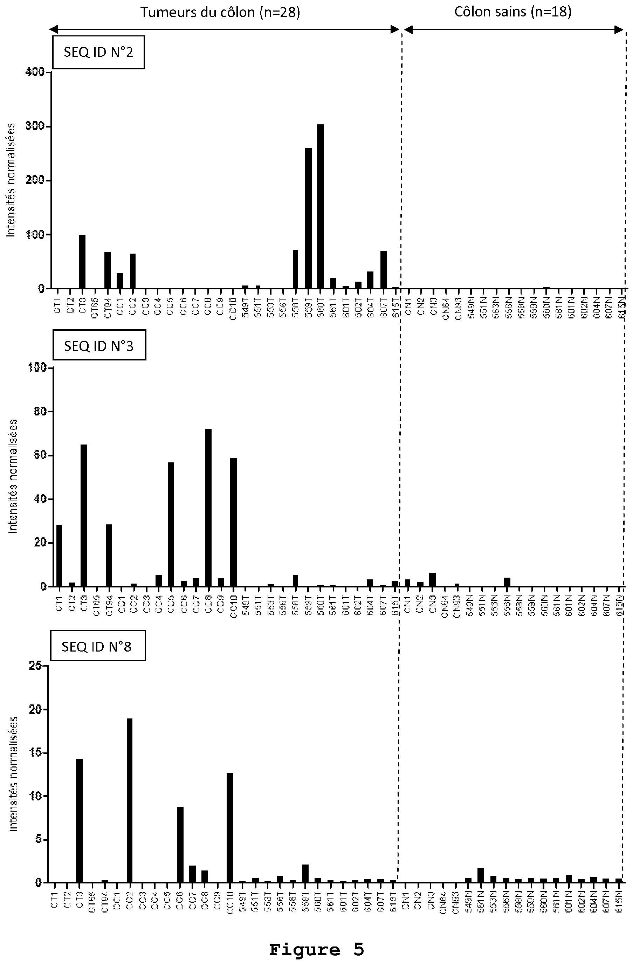

La figure 5 présente les résultats d'expression obtenus en RT-PCR quantitative sur les séquences SEQ ID N°2, SEQ ID N°3 et SEQ ID N°8, pour 28 tumeurs de côlon et 18 tissus sains de côlon, et met notamment en évidence l'intérêt de leur combinaison pour augmenter le nombre de détections au sein des échantillons de tumeurs colorectales.

### Exemples :

### Exemple 1 : Identification de séquences HERV présentant un différentiel d'expression dans le cancer du côlon

### Méthode :

L'identification de séquences HERV présentant un différentiel d'expression dans le cancer du côlon repose sur la conception et l'utilisation d'une puce à ADN haute densité au format GeneChip, dénommée HERV-V2, conçue par les inventeurs et dont la fabrication a été sous-traitée à la société Affymetrix. Cette puce contient des sondes qui correspondent à des séquences HERV distinctes au sein du génome humain. Ces séquences ont été identifiées à partir d'un ensemble de références prototypiques découpées en régions fonctionnelles (LTR, *gag, pol* et *env*) puis, par une recherche de similarité à l'échelle du génome humain entier (NCBI 36/hg18), 10 035 loci HERV distincts ont été identifiés, annotés, et finalement regroupés dans une banque de données nommée HERVgDB3.

Les sondes entrant dans la composition de la puce ont été définies à partir de HERVgDB3 et sélectionnées en appliquant un critère de spécificité d'hybridation, dont le but est d'exclure du procédé de création les sondes présentant un risque d'hybridation élevé avec une cible non recherchée. Pour cela, les séquences de HERVgDB3 ont d'abord été segmentées par ensemble de 25 nucléotides (25-mers) chevauchants, aboutissant à un ensemble de sondes candidates. Le risque d'hybridation aspécifique a ensuite été évalué pour chaque sonde candidate en réalisant des alignements sur l'ensemble du génome humain à l'aide de l'algorithme KASH (2). Un score expérimental sanctionne le résultat de l'hybridation, addition de l'impact du nombre, du type et de la position des erreurs dans l'alignement. La valeur de ce score est corrélée au potentiel d'hybridation cible/sonde. La connaissance de tous les potentiels d'hybridation d'une sonde candidate sur l'ensemble du génome humain permet d'évaluer sa spécificité de capture. Les sondes candidates qui présentent une bonne affinité de capture sont conservées puis regroupées en « probesets » et enfin synthétisées sur la puce HERV-V2.

Les échantillons analysés à l'aide de la puce haute densité HERV-V2 correspondent à des ARN extraits de tumeurs et aux ARN extraits des tissus sains adjacents à ces tumeurs. Les tissus analysés sont le côlon, avec en contrôles le sein, l'ovaire, l'utérus, la prostate, le poumon, le testicule et le placenta. Dans le cas du placenta, seuls des tissus sains ont été utilisés. Pour chaque échantillon, 50ng d'ARN ont servi à la synthèse de cDNA en utilisant le protocole d'amplification connu sous le nom de WTO. Le principe de l'amplification WTO est le suivant : des amorces aléatoires, ainsi que des amorces ciblant l'extrémité 3' du transcrit ARN, sont ajoutées, avant une étape de transcription inverse suivie d'une amplification linéaire et simple brin désignée SPIA. Les cDNA sont ensuite dosés, caractérisés et purifiés, puis 2µg sont fragmentés, marqués à la biotine en extrémité 3' par l'action de l'enzyme *terminal transferase.* Le produit cible ainsi préparé est mélangé à des oligonucléotides de contrôle, puis l'hybridation est réalisée selon le protocole recommandé par la société Affymetrix. Les puces sont alors révélées et lues pour acquérir l'image de leur fluorescence. Un contrôle qualité se basant sur les contrôles standards est réalisé, et un ensemble d'indicateurs (MAD, MAD-Med plots, RLE) servent à exclure les puces non conformes à une analyse statistique.

L'analyse des puces consiste d'abord en un prétraitement des données par l'application d'une correction du bruit de fond basée sur l'intensité des signaux des sondes tryptophanes, suivie d'une normalisation RMA (3) basée sur la méthode des quantiles. Puis une double correction des effets liés aux lots d'expériences est réalisée en appliquant la méthode COMBAT (4) afin de garantir que les différences d'expression observées sont d'origine biologique et non techniques. A ce stade, une analyse exploratoire des données est conduite à l'aide d'outils de regroupement de données par partitionnement euclidien (*clustering*), et enfin une analyse statistique utilisant la procédure SAM (5) suivie d'une correction par le taux de faux positif (6) et d'une suppression des valeurs inférieures à 2⁶ est appliquée pour la recherche de séquences présentant un différentiel d'expression entre l'état normal et l'état tumoral d'un tissu.

### Résultats :

Le traitement des données générées par l'analyse des puces à ADN HERV-V2 à l'aide de cette méthode a permis d'identifier un ensemble de « probesets » présentant une différence d'expression statistiquement significative entre le côlon normal et le côlon tumoral. Les résultats du *clustering* ainsi que la recherche d'expression différentielle au sein des échantillons contrôles a par ailleurs mis en évidence des éléments HERV dont l'expression différentielle est spécifiquement associée au côlon tumoral.

Les séquences nucléotidiques des éléments HERV présentant un différentiel d'expression dans le côlon tumoral sont identifiées par les SED ID N°1 à 285, la localisation chromosomique de chaque séquence est donnée dans le référentiel NCBI 36/hg18, et la mention « tissu cible » (une croix) pointe les éléments dont l'expression différentielle n'a été observée que dans la comparaison entre côlon normal et côlon tumoral (par rapport aux comparaisons au sein des tissus contrôles). Une valeur indicative du ratio d'expression entre état normal et état tumoral est également communiquée, et sert à ordonner les séquences dans un soucis de présentation uniquement.

### Exemple 2 : Détection de séquences HERV dans les fluides biologiques

### Principe :

Les inventeurs ont montré que des séquences HERV sont détectées dans les fluides biologiques, ce qui permet entre autres de caractériser un cancer du côlon par recours à une détection à distance de l'organe primaire. Une étude a été menée sur 20 échantillons d'urine et 38 échantillons de sérum provenant d'individus différents.

Les sérums et les urines ont été centrifugés dans les conditions suivantes :
Sérums : 500 g pendant 10 minutes à 4°C. Le surnageant a été récupéré et centrifugé de nouveau à 16500 g pendant 20 minutes à 4°C. Le surnageant de cette deuxième centrifugation, dépourvu de cellules, mais comprenant encore des exosomes, des microvésicules, des acides nucléiques, des protéines, a été analysé sur des puces. La puce est la puce HERV-V2 utilisée selon les modalités précédemment décrites.
Urines : après recueil, centrifugation à 800 g pendant 4 minutes à 4°C. Le culot a été récupéré avec du RNA protect cell reagent™. Puis, centrifugation à 5000 g pendant 5 minutes avant ajout du tampon de lyse sur le culot. La puce est la puce HERV-V2 utilisée selon les modalités précédemment décrites.

### Résultats :

Un nombre important de signaux positifs, incluant les signaux d'expression correspondant aux séquences listées dans le tableau ci-dessus, a été détecté à la fois dans les surnageants de sérums et dans les culots cellulaires provenant d'urines, comme illustré dans les figures 3 et 4. Ceci confirme que les fluides biologiques, en particulier le sérum et l'urine, sont une source utilisable de matériel biologique pour la détection de séquences HERV. Il est communément accepté que le seuil de positivité est de l'ordre de 2⁶ soit 64.

### Exemple 3 : mise en évidence de l'intérêt d'une combinaison de deux, et trois, séquences HERV pour la détection du cancer colorectal.

### Méthode :

Des ARN provenant de 28 tumeurs de côlon (CT1, CT2, CT3, CT65, CT94, CC1, CC2, CC3, CC4, CC5, CC6, CC7, CC8, CC9, CC10, 549T, 551T, 553T, 556T, 558T, 559T, 560T, 561T, 601T, 602T, 604T, 607T et 615T) et de 18 tissus sains de côlon (CN1, CN2, CN3, CN64, CN93, 549N, 551N, 553N, 556N, 558N, 559N, 560N, 561N, 601N, 602N, 604N, 607N et 615N) ont été extraits et utilisés pour apporter la démonstration que la combinaison des résultats d'expression de deux, ou trois, séquences HERV permet d'augmenter le nombre de détections au sein des échantillons de tumeurs colorectales.

Une amplification par RT-PCR quantitative correspondant aux régions 1723-1824 de la SEQ ID N°2, 1540-1665 de la SEQ ID N°3 et 1684-1923 de la SEQ ID N°8 a été réalisée pour l'ensemble des 46 échantillons de l'étude. Les résultats d'expression des séquences HERV ont été traités par la méthode du delta Ct et normalisés par les résultats d'expression du gène de ménage GAPDH. Les résultats sont présentés dans la figure 5.

### Résultats :

L'amplification par RT-PCR quantitative de la région 1723-1824 de la SEQ ID N°2 est positive pour 11 échantillons de tumeurs du côlon (CT3, CT94, CC1, CC2, 558T, 559T, 560T, 561T, 602T, 604T et 607T). Aucune détection de cette région n'est observée pour les échantillons de tissus sains de côlon.

L'amplification par RT-PCR quantitative de la région 1540-1665 de la SEQ ID N°3 est positive pour 6 échantillons de tumeurs du côlon (CT1, CT3, CT94, CC5, CC8 et CC10). Aucune détection de cette région n'est observée pour les échantillons de tissus sains de côlon.

L'amplification par RT-PCR quantitative de la région 1684-1923 de la SEQ ID N°8 est positive pour 4 échantillons de tumeurs du côlon (CT3, CC2, CC6 et CC10). Aucune détection de cette région n'est observée pour les échantillons de tissus sains de côlon.

La prise en compte additive des détections de la région 1723-1824 de la SEQ ID N°2 et de la région 1540-1665 de la SEQ ID N°3 permet une couverture de 15 échantillons de tumeurs de côlon (CT1, CT3, CT94, CC1, CC2, CC5, CC8, CC10, 558T, 559T, 560T, 561T, 602T, 604T et 607T), montrant ainsi un gain de sensibilité de cette combinaison de deux séquences HERV par rapport à l'utilisation de ces mêmes séquences HERV considérées isolément.

La prise en compte additive des détections de la région 1723-1824 de la SEQ ID N°2 et de la région 1684-1923 de la SEQ ID N°8 permet une couverture de 13 échantillons de tumeurs de côlon (CT3, CT94, CC1, CC2, CC6, CC10, 558T, 559T, 560T, 561T, 602T, 604T et 607T), montrant ainsi un gain de sensibilité de cette combinaison de deux séquences HERV par rapport à l'utilisation de ces mêmes séquences HERV considérées isolément.

La prise en compte additive des détections de la région 1540-1665 de la SEQ ID N°3 et de la région 1684-1923 de la SEQ ID N°8 permet une couverture de 8 échantillons de tumeurs de côlon (CT1, CT3, CT93, CC2, CC5, CC6, CC8 et CC10), montrant ainsi un gain de sensibilité de cette combinaison de deux séquences HERV par rapport à l'utilisation de ces mêmes séquences HERV considérées isolément.

Enfin, la prise en compte additive des détections de la région 1723-1824 de la SEQ ID N°2 et de la région 1540-1665 de la SEQ ID N°3 et de la région 1684-1923 de la SEQ ID N°8 permet une couverture de 16 échantillons de tumeurs de côlon (CT1, CT3, CT94, CC1, CC2, CC5, CC6, CC8, CC10, 558T, 559T, 560T, 561T, 602T, 604T et 607T), montrant ainsi un gain de sensibilité de cette combinaison de trois séquences HERV par rapport à l'utilisation de ces mêmes séquences HERV considérées isolément ou en combinaison deux à deux.

### Références bibliographiques

1. Nickerson,D.A., Taylor,S.L., Weiss,K.M., Clark,A.G., Hutchinson,R.G., Stengard,J., Salomaa,V., Vartiainen,E., Boerwinkle,E. and Sing,C.F. (1998) DNA sequence diversity in a 9.7-kb région of the human lipoprotein lipase gene. Nat. Genet., 19, 233-240.
2. Navarro,G. and Raffinot,M. (2002) Flexible Pattern Matching in Strings: Practical On-Line Search Algorithms for Texts and Biological Sequences. Cambridge University Press.
3. Irizarry,R.A., Hobbs,B., Collin,F., Beazer-Barclay,Y.D., Antonellis,K.J., Scherf,U. and Speed,T.P. (2003) Exploration, normalization, and summaries of high density oligonucleotide array probe level data. Biostatistics (Oxford, England), 4, 249-264.
4. Johnson,W.E., Li,C. and Rabinovic,A. (2007) Adjusting batch effects in microarray expression data using empirical Bayes methods. Biostatistics (Oxford, England), 8, 118-127.
5. Tusher,V.G., Tibshirani,R. and Chu,G. (2001) Significance analysis of microarrays applied to the ionizing radiation response. Proceedings of the National Academy of Sciences of the United States of America, 98, 5116-5121.
6. Storey,J.D. and Tibshirani,R. (2003) Statistical significance for genomewide studies. Proceedings of the National Academy of Sciences of the United States of America, 100, 9440-9445.

## Revendications

1. Procédé pour le diagnostic, in vitro, spécifique du cancer du côlon dans un échantillon biologique prélevé chez un patient qui comprend une étape de détection d'au moins un produit d'expression d'au moins une séquence d'acide nucléique choisie parmi les séquences identifiées en SEQ ID NOs : 1, 3, 4, 5, 7, 8, 9, 10, 11, 12, 13, 17, 19, 22, 24, 25, 27 et 97 ou parmi les séquences qui présentent au moins 99% d'identité avec une des séquences identifiées en SEQ ID NOs : 1, 3, 4, 5, 7, 8, 9, 10, 11, 12, 13, 17, 19, 22, 24, 25, 27 et 97.

2. Procédé selon la revendication 1, dans lequel est détecté le produit. d'expression d'au moins deux séquences d'acide nucléique, lesdites au moins deux séquences d'acide nucléique étant choisies dans le groupe de séquences identifiées en SEQ ID NOs : 1, 2, 3, 4, 5, 7, 8, 9,10,11,12,13,17,19, 22, 24, 25, 27 et 97 ou parmi les séquences qui présentent au moins 99% d'identité avec les séquences identifiées en SEQ ID NOs : 1, 2, 3, 4, 5, 7, 8, 9, 10, 11, 12, 13, 17, 19, 22, 24, 25, 27 et 97.

3. Procédé selon la revendication 2, dans lequel l'une des deux séquences est SEQ ID NO : 2 ou une séquence présentant au moins 99% d'identité avec SEQ ID NO : 2.

4. Procédé selon la revendication 2 ou 3, dans lequel est détecté le produit d'expression d'au moins deux séquences d'acide nucléique, de préférence de trois séquences d'acide nucléique, lesdites séquences d'acide nucléique étant choisies dans le groupe de séquences identifiées en SEQ ID NOs 2, 3 et 8, ou parmi les séquences qui présentent au moins 99% d'identité avec les séquences identifiées en SEQ ID NOs : 2, 3 et 8.

5. Procédé selon l'une quelconque des revendications 1 à 3, **caractérisé en ce qu'**on détecte, en outre, le produit d'expression d'au moins une séquence d'acide nucléique choisie parmi les séquences identifiées en SEQ ID NOs : 6, 14 à 16, 18, 20, 21, 23, 26, 28 à 96, 98 à 285 et les séquences qui présentent au moins 99% d'identité avec les séquences identifiées en SEQ ID NOs : 6, 14 à 16, 18, 20, 21, 23, 26, 28 à 96, 98 à 285.

6. Procédé selon l'une quelconque des revendications 1 à 5, dans lequel le produit d'expression détecté est au moins un transcrit ARN ou au moins un polypeptide.

7. Procédé selon la revendication 6, **caractérisé en ce que** le transcrit ARN est au moins un ARNm.

8. Procédé selon la revendication 6 ou 7, dans lequel le transcrit ARN, en particulier l'ARNm est détecté par hybridation, par amplification ou par séquençage.

9. Procédé selon la revendication 7, dans lequel l'ARNm est mis en contact avec au moins une sonde et/ou au moins une amorce dans des conditions prédéterminées permettant l'hybridation et en ce qu'on détecte la présence ou l'absence d'hybridation à l'ARNm.

10. Procédé selon la revendication 7, **caractérisé en ce qu'**on prépare des copies ADN de l'ARNm, on met en contact les copies ADN avec au moins une sonde et/ou au moins une amorce dans des conditions prédéterminées permettant l'hybridation et **en ce qu'**on détecte la présence ou l'absence d'hybridation aux dites copies ADN.

11. Procédé selon la revendication 6, dans lequel on détecte le polypeptide exprimé par mise en contact avec au moins un partenaire de liaison spécifique dudit polypeptide, en particulier un anticorps ou un analogue d'anticorps, une protéine d'affinité ou un aptamère.

12. Utilisation d'au moins une séquence d'acide nucléique, isolée, comme marqueur moléculaire pour le diagnostic, in vitro, spécifique du cancer du côlon, dans un échantillon biologique prélevé chez un patient, **caractérisée en ce que** la séquence d'acide nucléique consiste en
(i) au moins une séquence ADN choisie parmi les séquences identifiées en SEQ ID NOs : 1, 3, 4, 5, 7, 8, 9, 10, 11, 12, 13, 17, 19, 22, 24, 25, 27 et 97, ou
(ii) au moins une séquence ADN complémentaire d'au moins une séquence choisie parmi les séquences identifiées en SEQ ID NOs : 1, 3, 4, 5, 7, 8, 9, 10, 11, 12, 13, 17, 19, 22, 24, 25, 27 et 97, ou
(iii) au moins une séquence ADN qui présente au moins 99% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'au moins une séquence choisie parmi les séquences qui présentent au moins 99% « d'identité: avec: les séquences telles que définies en (i).

13. Utilisation selon la revendication 12 d'au moins deux séquences d'acide nucléique qui consistent en :
(i) au moins deux séquences d'ADN choisies parmi les séquences identifiées en SEQ ID NOs : 1, 2, 3, 4, 5, 7, 8, 9, 10, 11, 12, 13, 17, 19, 22, 24, 25, 27 et 97 et en particulier parmi les séquences identifiées en SEQ ID NOs : 2, 3 et 8, ou
(ii) au moins deux séquences d'ADN respectivement complémentaires d'au moins deux séquences choisies parmi les séquences identifiées en SEQ ID NOs : 1, 2, 3, 4, 5, 7, 8, 9, 10, 11, 12, 13, 17, 19, 22, 24, 25, 27 et 97 et en particulier parmi les séquences identifiées en SEQ ID NOs : 2, 3 et 8, ou
(iii) au moins deux séquences d'ADN qui présentent au moins 99% d'identité avec deux séquences telles que définies en (i) et (ii), ou
(iv) au moins deux séquences d'ARN qui sont respectivement le produit de transcription de deux séquences choisies parmi les séquences telles que définies en (i), ou
(v) au moins deux séquences d'ARN qui sont le produit de transcription d'au moins deux séquences choisies parmi les séquences qui présentent au moins 99% d'identité: avec les séquences telles que définies en (i).

14. Procédé pour évaluer l'efficacité d'un traitement du cancer du côlon qui comprend une étape de détection, dans un échantillon biologique prélevé sur un patient, d'au moins un produit d'expression d'au moins une séquence d'acide nucléique, ladite séquence d'acide nucléique étant choisie parmi les séquences identifiées en SEQ ID NOs : 1, 3, 4, 5, 7, 8, 9, 10, 11, 12, 13, 17, 19, 22, 24, 25, 27 et 97 ou parmi les séquences qui présentent au moins 99% d'identité respectivement avec les séquences identifiées en SEQ ID NOs :1, 3, 4, 5, 7, 8, 9, 10, 11, 12, 13, 17, 19, 22, 24, 25, 27 et 97.

15. Procédé selon la revendication 14, dans lequel on détecte le produit d'expression d'au moins deux séquences d'acide nucléique, lesdites au moins deux séquences d'acide nucléique étant choisies dans le groupe de séquences identifiées en SEQ ID NOs : 1, 2, 3, 4, 5, 7, 8, 9,10,11,12,13,17,19, 22, 24, 25, 27 et 97 ou parmi les séquences qui présentent au moins 99% d'identité avec les séquences identifiées en SEQ ID NOs : 1, 2, 3, 4, 5, 7, 8, 9, 10, 11, 12, 13, 17, 19, 22, 24, 25, 27 et 97.

16. Procédé selon la revendication 15, dans lequel l'une des deux séquences est SEQ ID NO : 2 ou une séquence présentant au moins 99% d'identité avec SEQ ID NO : 2.

17. Procédé selon la revendication 15 ou 16, dans lequel est détecté le produit d'expression d'au moins deux séquences d'acide nucléique, de préférence de trois séquences d'acide nucléique, lesdites séquences d'acide nucléiques étant choisies dans le groupe de séquences identifiées en SEQ ID NOs 2, 3 et 8, ou parmi les séquences qui présentent au moins 99% d'identité avec les séquences identifiées en SEQ ID NOs : 2, 3 et 8.

## Patentansprüche

1. Verfahren zur darmkrebsspezifischen In-vitro-Diagnose in einer von einem Patienten entnommenen biologischen Probe, das einen Schritt des Nachweisens von wenigstens einem Expressionsprodukt wenigstens einer Nukleinsäuresequenz umfasst, ausgewählt unter den Sequenzen, die in SEQ ID NOs : 1, 3, 4, 5, 7, 8, 9, 10, 11, 12, 13, 17, 19, 22, 24, 25, 27 und 97 identifiziert sind, oder unter den Sequenzen, die wenigstens 99 % Identität mit einer der Sequenzen aufweisen, die in SEQ ID NOs : 1, 3, 4, 5, 7, 8, 9, 10, 11, 12, 13, 17, 19, 22, 24, 25, 27 und 97 identifiziert sind.

2. Verfahren nach Anspruch 1, bei dem das Expressionsprodukt von wenigstens zwei Nukleinsäuresequenzen nachgewiesen wird, wobei die wenigstens zwei Nukleinsäuresequenzen ausgewählt sind aus der Gruppe von Sequenzen, die in SEQ ID NOs : 1, 2, 3, 4, 5, 7, 8, 9, 10, 11, 12, 13, 17, 19, 22, 24, 25, 27 und 97 identifiziert sind, oder unter den Sequenzen, die wenigstens 99 % Identität mit den Sequenzen aufweisen, die in SEQ ID NOs : 1, 2, 3, 4, 5, 7, 8, 9, 10, 11, 12, 13, 17, 19, 22, 24, 25, 27 und 97 identifiziert sind.

3. Verfahren nach Anspruch 2, bei dem eine der zwei Sequenzen SEQ ID NO : 2 oder eine Sequenz ist, die wenigstens 99 % Identität mit SEQ ID NO : 2 aufweist.

4. Verfahren nach Anspruch 2 oder 3, bei dem das Expressionsprodukt von wenigstens zwei Nukleinsäuresequenzen, bevorzugt von drei Nukleinsäuresequenzen nachgewiesen wird, wobei die Nukleinsäuresequenzen ausgewählt sind aus der Gruppe von Sequenzen, die in SEQ ID NOs : 2, 3 und 8 identifiziert sind, oder unter den Sequenzen, die wenigstens 99 % Identität mit den Sequenzen aufweisen, die in SEQ ID NOs : 2, 3 und 8 identifiziert sind.

5. Verfahren nach einem der Ansprüche 1 bis 3, **dadurch gekennzeichnet, dass** weiter das Expressionsprodukt von wenigstens einer Nukleinsäuresequenz nachgewiesen wird, ausgewählt unter den Sequenzen, die in SEQ ID NOs : 6, 14 bis 16, 18, 20, 21, 23, 26, 28 bis 96, 98 bis 285 identifiziert sind, und den Sequenzen, die wenigstens 99 % Identität mit den Sequenzen aufweisen, die in SEQ ID NOs : 6, 14 bis 16, 18, 20, 21, 23, 26, 28 bis 96, 98 bis 285 identifiziert sind.

6. Verfahren nach einem der Ansprüche 1 bis 5, bei dem das nachgewiesene Expressionsprodukt wenigstens ein RNA-Transkript oder wenigstens ein Polypeptid ist.

7. Verfahren nach Anspruch 6, **dadurch gekennzeichnet, dass** das RNA-Transkript wenigstens eine mRNA ist.

8. Verfahren nach Anspruch 6 oder 7, bei dem das RNA-Transkript, insbesondere die mRNA, durch Hybridisierung, durch Amplifizierung oder durch Sequenzierung nachgewiesen wird.

9. Verfahren nach Anspruch 7, bei dem die mRNA unter vorbestimmten Bedingungen, die die Hybridisierung ermöglichen, mit wenigstens einer Sonde und/oder wenigstens einem Initiator in Kontakt gebracht wird und dadurch, dass das Vorhandensein oder das Fehlen von Hybridisierung an der mRNA nachgewiesen wird.

10. Verfahren nach Anspruch 7, **dadurch gekennzeichnet, dass** DNA- und mRNA-Kopien hergestellt werden, die DNA-Kopien unter vorbestimmten Bedingungen, die die Hybridisierung ermöglichen, mit wenigstens einer Sonde und/oder wenigstens einem Initiator in Kontakt gebracht werden, und dadurch, dass das Vorhandensein oder das Fehlen von Hybridisierung an den DNA-Kopien nachgewiesen wird.

11. Verfahren nach Anspruch 6, bei dem das exprimierte Polypeptid durch Inkontaktbringen mit wenigstens einem spezifischen Bindungspartner des Polypeptids, insbesondere einem Antikörper oder einem Antikörper-Analogon, einem Affinitätsprotein oder einem Aptamer nachgewiesen wird.

12. Verwendung von wenigstens einer isolierten Nukleinsäuresequenz als molekularen Marker zur darmkrebsspezifischen In-vitro-Diagnose in einer von einem Patienten entnommenen biologischen Probe, **dadurch gekennzeichnet, dass** die Nukleinsäuresequenz besteht aus
(i) wenigstens einer DNA-Sequenz, ausgewählt unter den Sequenzen, die in SEQ ID NOs : 1, 3, 4, 5, 7, 8, 9, 10, 11, 12, 13, 17, 19, 22, 24, 25, 27 und 97 identifiziert sind, oder
(ii) wenigstens einer komplementären DNA-Sequenz von wenigstens einer Sequenz, ausgewählt unter den Sequenzen, die in SEQ ID NOs : 1, 3, 4, 5, 7, 8, 9, 10, 11, 12, 13, 17, 19, 22, 24, 25, 27 und 97 identifiziert sind, oder
(iii) wenigstens einer DNA-Sequenz, die wenigstens 99 % Identität mit einer Sequenz aufweist, wie in (i) und (ii) definiert, oder
(iv) wenigstens einer RNA-Sequenz, die das Transkriptionsprodukt einer Sequenz ist, ausgewählt unter den Sequenzen, wie in (i) definiert, oder
(v) wenigstens einer RNA-Sequenz, die das Transkriptionsprodukt von wenigstens einer Sequenz ist, ausgewählt unter den Sequenzen, die wenigstens 99 % Identität aufweisen mit: den Sequenzen, wie in (i) definiert.

13. Verwendung nach Anspruch 12 von wenigstens zwei Nukleinsäuresequenzen, die bestehen aus:
(i) wenigstens zwei DNA-Sequenzen, ausgewählt unter den Sequenzen, die in SEQ ID NOs : 1, 2, 3, 4, 5, 7, 8, 9, 10, 11, 12, 13, 17, 19, 22, 24, 25, 27 und 97 identifiziert sind, und insbesondere unter den Sequenzen, die in SEQ ID NOs : 2, 3 und 8 identifiziert sind, oder
(ii) wenigstens zwei DNA-Sequenzen, die jeweils mit wenigstens zwei Sequenzen komplementär sind, ausgewählt unter den Sequenzen, die in SEQ ID NOs : 1, 2, 3, 4, 5, 7, 8, 9, 10, 11, 12, 13, 17, 19, 22, 24, 25, 27 und 97 identifiziert sind, und insbesondere unter den Sequenzen, die in SEQ ID NOs : 2, 3 und 8 identifiziert sind, oder
(iii) wenigstens zwei DNA-Sequenzen, die wenigstens 99 % Identität mit zwei Sequenzen aufweisen, wie in (i) und (ii) definiert, oder
(iv) wenigstens zwei RNA-Sequenzen, die jeweils das Transkriptionsprodukt von zwei Sequenzen sind, ausgewählt unter den Sequenzen, wie in (i) definiert, oder
(v) wenigstens zwei RNA-Sequenzen, die das Transkriptionsprodukt von wenigstens zwei Sequenzen sind, ausgewählt unter den Sequenzen, die wenigstens 99 % Identität aufweisen: mit den Sequenzen, wie in (i) definiert.

14. Verfahren zur Beurteilung der Wirksamkeit einer Behandlung des Darmkrebses, die einen Schritt des Nachweisens, in einer von einem Patienten entnommenen biologischen Probe, von wenigstens einem Expressionsprodukt wenigstens einer Nukleinsäuresequenz umfasst, wobei die Nukleinsäuresequenz ausgewählt ist unter den Sequenzen, die in SEQ ID NOs : 1, 3, 4, 5, 7, 8, 9, 10, 11, 12, 13, 17, 19, 22, 24, 25, 27 und 97 identifiziert sind, oder unter den Sequenzen, die wenigstens 99 % Identität jeweils mit den Sequenzen aufweisen, die in SEQ ID NOs : 1, 3, 4, 5, 7, 8, 9, 10, 11, 12, 13, 17, 19, 22, 24, 25, 27 und 97 identifiziert sind.

15. Verfahren nach Anspruch 14, bei dem das Expressionsprodukt von wenigstens zwei Nukleinsäuresequenzen nachgewiesen wird, wobei die wenigstens zwei Nukleinsäuresequenzen ausgewählt sind aus der Gruppe von Sequenzen, die in SEQ ID NOs :1, 2, 3, 4, 5, 7, 8, 9, 10, 11, 12, 13, 17, 19, 22, 24, 25, 27 und 97 identifiziert sind, oder unter den Sequenzen, die wenigstens 99 % Identität mit den Sequenzen aufweisen, die in SEQ ID NOs : 1, 2, 3, 4, 5, 7, 8, 9, 10, 11, 12, 13, 17, 19, 22, 24, 25, 27 und 97 identifiziert sind.

16. Verfahren nach Anspruch 15, bei dem eine der zwei Sequenzen SEQ ID NO : 2 ist oder eine Sequenz, die wenigstens 99 % Identität mit SEQ ID NO : 2 aufweist.

17. Verfahren nach Anspruch 15 oder 16, bei dem das Expressionsprodukt von wenigstens zwei Nukleinsäuresequenzen, bevorzugt von drei Nukleinsäuresequenzen nachgewiesen wird, wobei die Nukleinsäuresequenzen ausgewählt sind aus der Gruppe von Sequenzen, die in SEQ ID NOs : 2, 3 und 8 identifiziert sind, oder unter den Sequenzen, die wenigstens 99 % Identität mit den Sequenzen aufweisen, die in SEQ ID NO : 2, 3 und 8 identifiziert sind.

## Claims

1. A method for the *in vitro* diagnosis specific to colon cancer in a biological sample taken from a patient which comprises a step of detecting at least one expression product of at least one nucleic acid sequence selected from the sequences identified in SEQ ID NOs: 1, 3, 4, 5, 7, 8, 9, 10, 11, 12, 13, 17, 19, 22, 24, 25, 27 and 97 or from the sequences which have at least 99% of identity with one of the sequences identified in SEQ ID NOs: 1, 3, 4, 5, 7, 8, 9, 10, 11, 12, 13, 17, 19, 22, 24, 25, 27 and 97.

2. The method according to claim 1, wherein the expression product of at least two nucleic acid sequences is detected, said at least two nucleic acid sequences being selected from the group of sequences identified in SEQ ID NOs: 1, 2, 3, 4, 5, 7, 8, 9, 10 , 11, 12, 13, 17, 19, 22, 24, 25, 27 and 97 or from the sequences which have at least 99% of identity with the sequences identified in SEQ ID NOs: 1, 2, 3, 4, 5, 7, 8, 9, 10, 11, 12, 13, 17, 19, 22, 24, 25, 27 and 97.

3. The method according to claim 2, wherein one of the two sequences is SEQ ID NO: 2 or a sequence having at least 99% of identity with SEQ ID NO: 2.

4. The method according to claim 2 or 3, wherein the expression product of at least two nucleic acid sequences, preferably of three nucleic acid sequences, is detected, said nucleic acid sequences being selected from the group of sequences identified in SEQ ID NOs 2, 3 and 8, or from the sequences which have at least 99% of identity with the sequences identified in SEQ ID NOs: 2, 3 and 8.

5. The method according to any one of claims 1 to 3, **characterized in that** the expression product of at least one nucleic acid sequence selected from the sequences identified in SEQ ID NOs: 6, 14 to 16, 18, 20, 21, 23, 26, 28 to 96, 98 to 285 and the sequences which have at least 99% of identity with the sequences identified in SEQ ID NOs: 6, 14 to 16, 18, 20, 21, 23, 26, 28 to 96, 98 to 285 are further detected.

6. The method according to any one of claims 1 to 5, wherein the detected expression product is at least one RNA transcript or at least one polypeptide.

7. The method according to claim 6, **characterized in that** the RNA transcript is at least one mRNA.

8. The method according to claim 6 or 7, wherein the RNA transcript, in particular the mRNA is detected by hybridization, amplification or sequencing.

9. The method according to claim 7, wherein the mRNA is brought into contact with at least one probe and/or at least one primer under predetermined conditions allowing the hybridization and the presence or the absence of hybridization with mRNA to be detected.

10. The method according to claim 7, **characterized in that** DNA copies of the mRNA are prepared, the DNA copies are brought into contact with at least one probe and/or at least one primer under predetermined conditions allowing the hybridization and the presence or the absence of hybridization with said DNA copies to be detected.

11. The method according to claim 6, wherein the expressed polypeptide is detected by bringing into contact with at least one specific binding partner of said polypeptide, in particular an antibody or an antibody analog, an affinity protein or an aptamer.

12. A use of at least one isolated nucleic acid sequence as a molecular marker for the *in vitro* diagnosis specific to colon cancer, in a biological sample taken from a patient, **characterized in that** the nucleic acid sequence consists of
(i) at least one DNA sequence selected from the sequences identified in SEQ ID NOs: 1, 3, 4, 5, 7, 8, 9, 10, 11, 12, 13, 17, 19, 22, 24, 25, 27 and 97, or
(ii) at least one DNA sequence complementary to at least one sequence selected from the sequences identified in SEQ ID Nos. 1, 3, 4, 5, 7, 8, 9, 10, 11, 12, 13, 17, 19, 22, 24, 25, 27 and 97, or
(iii) at least one DNA sequence which has at least 99% of identity with a sequence as defined in (i) and (ii), or
(iv) at least one RNA sequence which is the transcription product of a sequence selected from the sequences as defined in (i), or
(v) at least one RNA sequence which is the transcription product of at least one sequence selected from the sequences which have at least 99% of identity with the sequences as defined in (i).

13. The use according to claim 12 of at least two nucleic acid sequences which consist of:
(i) at least two DNA sequences selected from the SEQ ID NOs: 1, 2, 3, 4, 5, 7, 8, 9, 10, 11, 12, 13, 17, 19, 22, 24, 25, 27 and 97 and in particular from the sequences identified in SEQ ID NOs: 2, 3 and 8, or
(ii) at least two DNA sequences respectively complementary to at least two sequences selected from the sequences identified in SEQ ID NOs: 1, 2, 3, 4, 5, 7, 8, 9, 10, 11, 12, 13, 17, 19, 22, 24, 25, 27 and 97 and in particular from the sequences identified in SEQ ID NOs: 2, 3 and 8, or
(iii) at least two DNA sequences which have at least 99% of identity with two sequences as defined in (i) and (ii), or
(iv) at least two RNA sequences which are respectively the transcription product of two sequences selected from the sequences as defined in (i), or
(v) at least two RNA sequences which are the transcription product of at least two sequences selected from the sequences which have at least 99% identity with the sequences as defined in (i).

14. A method for evaluating the efficiency of a colon cancer treatment which comprises a step of detecting, in a biological sample taken from a patient, at least one expression product of at least one nucleic acid sequence, said nucleic acid sequence being selected from the sequences identified in SEQ ID NOs: 1, 3, 4, 5, 7, 8, 9, 10, 11, 12, 13, 17, 19, 22, 24, 25, 27 and 97 or from the sequences which have at least 99% of identity respectively with the sequences identified in SEQ ID NOs: 1, 3, 4, 5, 7, 8, 9, 10, 11, 12, 13, 17, 19, 22, 24, 25, 27 and 97.

15. The method according to claim 14, wherein the expression product of at least two nucleic acid sequences is detected, said at least two nucleic acid sequences being selected from the group of sequences identified in SEQ ID NOs: 1, 2, 3, 4, 5, 7, 8, 9, 10, 11, 12, 13, 17, 19, 22, 24, 25, 27 and 97 or from the sequences which have at least 99% of identity with the sequences identified in SEQ ID NOs: 1, 2, 3, 4, 5, 7, 8, 9, 10, 11, 12, 13, 17, 19, 22, 24, 25, 27 and 97.

16. The method according to claim 15, wherein one of the two sequences is SEQ ID NO: 2 or a sequence having at least 99% of identity with SEQ ID NO: 2.

17. The method according to claim 15 or 16, wherein the expression product of at least two nucleic acid sequences, preferably of three nucleic acid sequences, is detected, said nucleic acid sequences being selected from the group of sequences identified in SEQ ID NOs: 2, 3 and 8, or from the sequences which have at least 99% of identity with the sequences identified in SEQ ID Nos: 2, 3 and 8.
